Europäisches Patentamt

European Patent Office

Office européen des brevets

(11) Numéro de publication : **0 414 605 B1**

(12) **FASCICULE DE BREVET EUROPEEN**

(45) Date de publication du fascicule du brevet :
26.05.93 Bulletin 93/21

(51) Int. Cl.$^5$ : **A61K 45/06,** A61K 33/30,
A61K 7/48

(21) Numéro de dépôt : **90402327.2**

(22) Date de dépôt : **22.08.90**

(54) Compositions pharmaceutiques de type pâtes à l'eau.

(30) Priorité : **23.08.89 FR 8911172**

(43) Date de publication de la demande :
27.02.91 Bulletin 91/09

(45) Mention de la délivrance du brevet :
26.05.93 Bulletin 93/21

(84) Etats contractants désignés :
BE CH DE FR GB IT LI LU NL

(56) Documents cités :
US-A- 1 809 082
SOAP, PERFUMERY & COSMETICS, vol. 54,
no. 11, novembre 1981, London (GB); V.KIN-
GLAKE, pp. 607-613.
CHEMICAL ABSTRACTS, vol. 93, no. 16, octobre 1980, Columbus, OH (US); p. 355, no.
155864d.

(73) Titulaire : ROUSSEL-UCLAF
35, Boulevard des Invalides
F-75007 Paris (FR)

(72) Inventeur : Enjolras, Odile
8, Impasse A. Guilmart
F-92190 Meudon (FR)
Inventeur : Noel, Hugues
28, rue du Général Lherillier
F-95120 Ermont (FR)

(74) Mandataire : Bourgouin, André et al
Département des Brevets ROUSSEL UCLAF
111, route de Noisy B.P. no 9
F-93230 Romainville (FR)

## Description

La présente invention concerne des compositions pharmaceutiques de type "pâtes à l'eau".

Les pâtes à l'eau sont des préparations dermopharmaceutiques à base d'oxyde de zinc, l'oxyde de zinc favorisant la cicatrisation, destinées à soigner les irritations provoquées par la macération des selles et de l'urine dans les couches des nouveaux-nés.

La peau du nouveau-né ressemble beaucoup par sa structure à la peau de l'adulte, si l'enfant est à terme, les prématurés ayant une couche cornée n'assurant pas encore totalement sa fonction de barrière. Normalement le pH de la peau est acide, de 4 à 5,5. La peau est capable de contrer et de subir les agressions extérieures chez l'enfant comme chez l'adulte.

Mais chez les nouveaux-nés, les couches peuvent créer un environnement plus dur que celui habituellement rencontré par la peau, provoquant un risque de dermatite : le derme est agressé, la peau est irritée, enflammée.

Le principal effecteur dans l'irritation qui caractérise la dermatite des couches ou érythème fessier est le mélange urines-selles.

En effet, l'urée contenue dans l'urine est dégradée en ammoniaque par les uréases ce qui induit une augmentation de pH. Lorsque le pH devient basique, les enzymes fabriquées lors de la digestion telles que les protéases et les lipases d'origine pancréatique ou intestinale, voient leur activité donc leur pouvoir irritant augmenter, les lipases attaquant en particulier les triglycérides du sébum et provoquant la libération d'acides gras.

La couche cornée rendue perméable par une hyperhydratation, un frottement important, une digestion par les enzymes, perd sa fonction de barrière et laisse passer d'autres molécules irritantes telles que les sels biliaires.

On a pu observer dans certains cas une véritable digestion de l'épiderme du siège de l'enfant due à l'action d'uréases, de lipases et de protéases.

Pour lutter contre l'érythème fessier, les moyens de la thérapie actuelle, tels que la surveillance du régime alimentaire de l'enfant et du type de lange utilisé, l'assurance d'une bonne hygiène, l'application de pâtes à l'eau classiques, ne sont pas jugés satisfaisants par un grand nombre de dermatologues et de pédiatres.

L'article référencé comme suit : Soap, Perfumery & Cosmetics, vol. 54 (1981) Nov., n° 11, pp 607-613 décrit des compositions destinées à la peau d'enfant en bas âge, cependant la présence d'anti enzymes n'est pas indiquée dans ces compositions.

Or il vient d'être montré, et c'est l'objet de la présente invention, que l'association d'antienzymes dans une pâte à l'eau permettait de soigner les irritations provoquées par les selles et de diminuer considérablement les actions lipolytiques, protéolytiques et uréasiques, causes de l'irritation.

L'invention a pour objet de nouvelles compositions pharmaceutiques caractérisées en ce qu'elles renferment de l'oxyde de zinc et une ou plusieurs antienzymes, choisies parmi les antilipases, les antiprotéases et les antiuréases, elles-mêmes choisies parmi

a) les esters d'alcool gras choisirs parmi les acétate, lactate ou propionate d'alkyle renfermant de 10 à 20 atomes de carbone saturé ou insaturé, linéaire ou ramifié,

b) l'acide phytique, l'acide nitrilotriacétique, l'acide éthylène diamino tétracétique, l'acide diéthylène triamino pentacétique ou l'acide hydroxyéthyl éthylène diamino triacétique ainsi que les sels d'acides correspondants,

c) un sel de zinc d'acide gras choisi parmi les acides propionique, isobutyrique, caproïque et undécylénique,

d) un sel de zinc d'acide aminé acylé de formule :

$$R1 - CO - NH - R2$$

dans laquelle :

R1 - CO - est un groupement acyle dérivé d'un acide gras contenant de 2 à 22 atomes de carbone saturé ou insaturé, linéaire ou ramifié,

et - NH - R2 est un radical dérivé d'un acide aminé naturel ou d'un hydrolysat de protéine.

Les antienzymes sont des inhibiteurs d'enzymes dont le mode d'action peut être de types divers.

Un tel exemple d'inhibition est celui provoqué par des agents chélatants qui piègent des métaux indispensables à l'activité de l'enzyme.

Parmi ces agents chélatants, on trouve, l'acide phytique mais, également l'acide nitrilotriacétique, l'acide éthylène diamino tétracétique, l'acide diéthylène triamino pentacétique ou encore l'acide hydroxyéthyl éthylène diamino triacétique ainsi que les sels d'acides correspondants.

Ces agents chélatants peuvent être utilisés, de préférence, à une concentration comprise entre 0,1 et 2 %.

Ces compositions sont destinées spécialement au soin des érythèmes fessiers chez le nouveau-né et ont une double action curative et préventive.

Les compositions selon l'invention peuvent comporter des antienzymes d'une, de deux ou des trois catégories enzymatiques indiquées.

Par ailleurs, pour chacune des catégories enzymatiques envisagées, on peut utiliser un ou plusieurs représentants d'antienzymes.

L'invention a donc particulièrement pour objet de nouvelles compositions pharmaceutiques telles que définies ci-dessus caractérisées en ce qu'elles renferment une antilipase qui est un ester d'alcool gras.

On choisira comme antilipase de préférence des esters d'alcool gras tels que l'on peut trouver, par exemple, dans le brevet 2 305 172.

Ces esters d'alcool gras peuvent être, de préférence à une concentration comprise entre 0,1 et 5 %.

Une liste, d'exemples de telles antiprotéases peut être donnée ci-dessous.

Dans les sels de zinc d'acide aminé acylé de formule :

$$R1 - CO - NH - R2$$

le groupement R1 - CO - peut représenter, par exemple, le groupement acyl dérivé des acides propionique, butyrique, caproïque, undécylénique ou palmitique,

et le groupement - NH - R2 représente :

soit un radical dérivé d'un acide aminé naturel choisi parmi la glycine, l'alanine, la valine, la leucine, l'isoleucine, la sérine, la thréonine, la cystéine, la méthionine, la lysine, l'arginine, l'acide aspartique, l'acide glutamique, la phénylalanine, la tyrosine, l'histidine, la proline et l'ornithine,

soit un radical dérivé d'un hydrolysat de protéine tel que, par exemple, le collagène, la gélatine, la soie, la kératine de laine, plume ou crin, le soja, le blé (gluten) ou le maïs.

Ainsi, comme sels de zinc d'acide aminé acylé de formule :

$$R1 - CO - NH - R2$$

on peut citer, par exemple, la propionylcystéine, la propionylhydroxyproline ou le caproylcystéine (LIFACIDE [R]).

De préférence, l'antiprotéase choisie est l'acide phytique, également utilisé comme antiuréase.

La concentration préférentielle d'acide phytique est comprise entre 0,1 et 2 % dans les préparations telles que définies ci-dessus.

Les compositions pharmaceutiques selon l'invention peuvent se présenter sous toutes les formes utilisées en dermatologie, plus particulièrement de manière à pouvoir être administrées par voie locale : solutions, émulsions, crèmes, pommades, poudres, lait, lotion ou gel conditionnés, selon le cas, en pot ou en tube, en flacon de verre ou de plastique et éventuellement en flacon doseur ou encore en ampoules.

Les formes pharmaceutiques sont préparées selon les méthodes usuelles.

Pour chaque forme, on a recours à des excipients appropriés. Ces excipients doivent avoir toutes les qualités habituellement requises. Dans le cas d'une administration locale, ils doivent être doués d'une grande affinité pour la peau, être parfaitement bien tolérés, stables, présenter une consistance adéquate permettant une utilisation facile et agréable.

A titre d'exemples d'excipients pouvant être utilisés, on peut citer des polymères de type carboxyvinylique, les polyéthylèneglycols, le propylèneglycol, des cires, des corps gras, des esters et des triglycérides d'acides gras, des dérivés stéariques tel que, par exemple, le stéarate de glycérol, des alcools tels que, par exemple, les alcools stéaryliques, les alcools cétostéaryliques, l'alcool cétylique, polyol, l'éther cétylique polyoxyéthylène, des huiles végétales telles que l'huile d'amande douce, des huiles minérales telles que l'huile de vaseline, la glycérine, des dérivés de la lanoline, du talc, des mouillants, des épaississants, des stabilisants, des émulsionnants, des conservateurs, des parfums, des colorants ou d'autres excipients connus et couramment utilisés.

Les compositions cosmétiques peuvent être préparées en utilisant les mêmes ingrédients. Les doses peuvent être adaptées si nécessaire.

Les formes préférées sont les compositions pharmaceutiques telles que définies ci-dessus caractérisées en ce qu'elles se présentent sous forme de gel ou de crème.

L'invention a également pour objet de nouvelles compositions cosmétiques destinées notamment au soin des érythèmes fessiers chez le nouveau-né caractérisées en ce qu'elles renferment de l'oxyde de zinc et une ou plusieurs antienzymes.

Les compositions cosmétiques peuvent être préparées en utilisant les mêmes ingrédients que les compositions pharmaceutiques. Les doses peuvent être adaptées si nécessaire.

L'invention concerne ainsi notamment l'application à titre de produit cosmétique des compositions telles que définies ci-dessus.

L'invention concerne tout particulièrement une méthode de traitement cosmétique des érythèmes fessiers chez le nouveau-né

caractérisée en ce que l'on utilise les compositions cosmétiques telles que définies ci-dessus.

3

Les différentes formes pharmaceutiques et cosmétiques mentionnées ci-dessus peuvent être obtenues selon les méthodes usuelles utilisées dans ce domaine.

L'exemple suivant illustre l'invention sans toutefois la limiter.

**EXEMPLE 1** :

| | |
|---|---|
| Oxyde de zinc | 20 |
| Oxyde de titane | 3 |
| Talc | 10 |
| Hectorite | 1 |
| Silicate d'aluminium | 5 |
| Silice | 2 |
| Hydroxyéthylcellulose | 0,5 |
| Polyglycol | 10 |
| Glycérine | 15 |
| Butanediol 1,3 | 5 |
| D panthénol | 2 |
| Acétate d'oleyle | 1 |
| Acide phytique | 1 |
| Propionyl hydroxyproline | 1 |
| Caproylcystéine | 1 |
| Palmitate d'acide aminés de collagène | 0,5 |
| Eau | qsp 100 |

**Exemple 2 : Pâte à l'eau**

| | |
|---|---|
| Oxyde de zinc | 15 |
| Oxyde de titane | 3 |
| Talc | 10 |
| Silicate d'ammonium | 7 |
| Silice | 2,5 |
| Hydroxyéthyl cellulose | 0,2 |
| Polyglycol | 10 |
| Glycérine | 20 |
| Butanediol 1,3 | 2 |
| D-Panthénol | 2 |

```
Acétate d'oléyle                                        1
Acide phytique                                          1
Condensat d'acide caprylique et de cystéine 1
Condensat d'acide palmitique et de caséine  0,3
Conservateurs                                          qs
Eau                                                    qs 100
```

## Test d'activité antiuréasique, in vitro, de l'acide phytique

Les conditions opératoires communes aux quatre expériences menées en parallèle soit 1) 2) 3) et 4), dont les résultats sont indiqués dans le tableau ci-dessous, sont un mélange de substrat ou urée à 0,17 mole/l et d'enzyme ou uréase à 2 mg/ml (MERCK [R]) maintenu à 30°C dans un bain thermostaté.

L'expérience 1) est menée en absence d'inhibiteur tandis que les expériences 2), 3) et 4) sont réalisées en présence respectivement :
- pour 2) de thiourée à 0,17 mole/l, inhibiteur spécifique de l'uréase,
- pour 3) d'acide phytique à 0,425 mole/l en 3a) et 0,425 mmole/l en 3b),
- pour 4) de la pâte à l'eau de l'exemple 1.

Le tableau de résultats ci-dessous montre que :

1 - par comparaison des expériences 2) et 3) que l'acide phytique est un meilleur inhibiteur que la thiourée,

2 - par comparaison dans l'expérience 3) de 3a) et 3b) que l'acide phytique est actif à faible dose soit 0,425 mmole/l,

3 - par comparaison de l'expérience 3b) et de l'expérience 4) où la quantité d'acide phytique dans la pâte à l'eau est environ de 0,425 mmoles/l que la pâte à l'eau constitue un excellent inhibiteur assurant une bonne stabilité du pH.

| Temps | 1) Pas d'inhibiteur | 2) Thiourée 0,17 m/l | 3) Acide phytique 3a) | 3) Acide phytique 3b) | 4) Pâte à l'eau |
|---|---|---|---|---|---|
| 5 s | 6,17 | 6,25 | 6,08 | 6,13 | 6,68 |
| 10 s | 6,68 | 6,76 | – | 6,15 | – |
| 20 s | 7,77 | 7,85 | – | 6,15 | – |
| 40 s | 8,30 | 8,31 | – | 6,15 | – |
| 1 mn | 8,40 | 8,41 | 6,08 | – | 6,68 |
| 2 mn | 8,52 | 8,54 | 6,08 | 6,17 | 6,67 |
| 3 mn | 8,57 | 8,58 | 6,08 | 6,18 | 6,68 |
| 4 mn | 8,60 | – | 6,08 | 6,20 | 6,70 |

## Revendications

1. Compositions pharmaceutiques caractérisées en ce qu'elles renferment de l'oxyde de zinc et une ou plusieurs antienzymes, choisies parmi les antilipases, les antiprotéases et les antiuréases, elles-mêmes choisies parmi

a) les esters d'alcool gras choisis parmi les acétate, lactate ou propionate d'alkyle renfermant de 10 à 20 atomes de carbone saturé ou insaturé, linéaire ou ramifié,

b) l'acide phytique, l'acide nitrilotriacétique, l'acide éthylène diamino tétracétique, l'acide diéthylène triamino pentacétique ou l'acide hydroxyéthyl éthylène diamino triacétique ainsi que les sels d'acides correspondants,

c) un sel de zinc d'acide gras choisi parmi les acides propionique, isobutyrique, caproïque et undécylénique,

d) un sel de zinc d'acide aminé acylé de formule :

$$R1 - CO - NH - R2$$

dans laquelle :

R1 - CO - est un groupement acyle dérivé d'un acide gras contenant de 2 à 22 atomes de carbone saturé ou insaturé, linéaire ou ramifié,

et - NH - R2 est un radical dérivé d'un acide aminé naturel ou d'un hydrolysat de protéine.

2. Compositions pharmaceutiques telles que définies à la revendication 1, caractérisées en ce qu'elles se présentent sous forme de gel ou de crème.

3. Compositions cosmétiques destinées notamment au soin des érythèmes fessiers chez le nouveau-né, caractérisées en ce qu'elles renferment de l'oxyde de zinc et une ou plusieurs antienzymes, telles que définies à la revendication 1.

4. Application à titre de produit cosmétique des compositions telles que définies à la revendication 3.


**Patentansprüche**

1. Pharmazeutische Zusammensetzungen, dadurch gekennzeichnet, daß sie Zinkoxid und eines oder mehrere Antienzyme, ausgewählt unter den Antilipasen, den Antiproteasen und den Antiureasen, enthalten, die ihrerseits ausgewählt sind unter

a) den Fettalkohol-estern, ausgewählt unter den Alkylacetaten, -lactaten oder -propionaten mit linearem oder verzweigtem, gesättigtem oder ungesättigtem Alkyl mit 10 bis 20 Kohlenstoffatomen,

b) der Phytinsäure, der Nitrilo-triessigsäure, der Ethylendiamino-tetraessigsäure, der Diethylentriamino-pentaessigsäure, der Hydroxyethylethylendiamino-triessigsäure sowie den entsprechenden Säuresalzen,

c) einem Zinksalz einer Fettsäure, ausgewählt unter der Propion-, Isobutter-, Capron- und Undecylensäure,

d) einem Zinksalz einer acylierten Aminosäure der Formel

$$R1 - CO - NH - R2$$

worin R1 - CO - für eine Acylgruppe steht, die sich von einer linearen oder verzweigten, gesättigten oder ungesättigten Fettsäure mit 2 bis 22 Kohlenstoffatomen ableitet,

und - NH - R2 für einen Rest steht, der sich von einer natürlichen Aminosäure oder einem Proteinhydrolysat ableitet.

2. Pharmazeutische Zusammensetzungen, wie in Anspruch 1 definiert, dadurch gekennzeichnet, daß sie in Form eines Gels oder einer Creme vorliegen.

3. Kosmetische Zusammensetzungen, insbesondere für die Behandlung von Erythemen am Gesäß bei Neugeborenen, dadurch gekennzeichnet, daß sie Zinkoxid und eines oder mehrere Antienzyme, wie in Anspruch 1 definiert, enthalten.

4. Verwendung der Zusammensetzungen, wie in Anspruch 3 definiert, als kosmetisches Produkt.


**Claims**

1. Pharmaceutical compositions characterized in that they contain zinc oxide and one or more antienzymes, chosen from antilipases, antiproteases and antiureases, themselves chosen from

a) fatty alcohol esters chosen from saturated or unsaturated, linear or branched alkyl acetate, lactate or propionate containing 10 to 20 carbon atoms,

b) phytic acid, nitrilotriacetic acid, ethylene diamino tetracetic acid, diethylene triamino pentacetic acid or hydroxyethyl ethylene diamino triacetic acid as well as the corresponding acid salts,
c) a fatty acid zinc salt chosen from propionic, isobutyric, caproic and undecylenic acids,
d) an acylated amino acid zinc salt of formula:

$$R1 - CO - NH - R2$$

in which:

R1 - CO - is a saturated or unsaturated, linear or branched acyl group derived from a fatty acid containing 2 to 22 carbon atoms,

and - NH - R2 is a radical derived from a natural amino acid or a protein hydrolyzate.

2. Pharmaceutical compositions as defined in claim 1, characterized in that they are presented in the form of a gel or a cream.

3. Cosmetic compositions intended in particular for treating gluteal erythema in new-born babies, characterized in that they contain zinc oxide and one or more antienzymes, as defined in claim 1.

4. Use as cosmetic products of the compositions as defined in claim 3.